# EUROPEAN PATENT APPLICATION

(11) **EP 3 124 065 A1**
(43) Date of publication of application: **01.02.2017**
(21) Application number: 15768209.7
(22) Date of filing: 16.03.2015
(51) Int. Cl.: A61M 5/142, A61M 5/172

(54) **MEDICAL PUMP MONITORING DEVICE AND MEDICAL PUMP MONITORING METHOD**

(30) Priority: 27.03.2014 JP 2014066553
(71) Applicant: Terumo Kabushiki Kaisha, Tokyo 151-0072 (JP)
(72) Inventor: SUGAWARA, Hideki, Ashigarakami-gun Kanagawa 259-0151 (JP)
(74) Representative: KIPA AB
(86) International application number: PCT/JP2015/057640
(87) International publication number: WO 2015/146663

(57) **Abstract**

There is provided a medical pump monitoring device configured to manage a plurality of medical pumps arranged bedside, including: a reading terminal configured to sequentially read pump identification information items provided to the medical pumps; and an arithmetic processing unit configured to create image data used to display pump information items associated with the pump identification information items as a layout that maintains reading order in which the reading terminal reads the pump identification information items.

## Description

### Technical Field

The present invention relates to a medical pump monitoring device and a medical pump monitoring method, and in particular relates to a medical pump monitoring device and a medical pump monitoring method suitably used to manage a plurality of medical pumps for medical treatment in which the medical pumps are used for a patient.

### Background Art

Medical treatment in which a plurality of medical pumps is used for a patient is given in medical practice. Such medical treatment requires intensive management of the pump operation of the medical pumps in accordance with the pump information items collected from the medical pumps. In light of the foregoing, a system is proposed (e.g., refer to Patent Literature 1), and the system processes the data collected from a plurality of pumps, identifies the simultaneously operating pumps as a display image, and individually displays the state information items of the simultaneously operating pumps.

This system enables, for example, intensive detection of the information items of a plurality of pumps authenticated for the patient on a display image.

### Citation List

### Patent Literature

Patent Literature 1: JP 2005-28102 A

### Summary of Invention

### Technical Problem

However, the system described above is configured to display the information items of the pumps as a list on a display image. This configuration makes it difficult to immediately identify which pump is in an abnormal drive state among the pumps actually placed bedside of the patient when it is detected on the display image that one of the pumps is in an abnormal drive state. This may be a consequence of a medical error during an emergency response.

In light of the foregoing, an objective of the present invention is to provide a medical pump monitoring device and a medical pump monitoring method that can easily link the pump information items on the display image to the pumps actually placed bedside.

### Solution to Problem

A medical pump monitoring device of the present invention to achieve the above object includes: a reading terminal configured to sequentially read pump identification information items provided to the medical pumps; and an arithmetic processing unit configured to create image data used to display pump information items associated with the pump identification information items as a layout that maintains reading order in which the reading terminal reads the pump identification information items.

A medical pump monitoring method of the present invention includes: a reading step of reading pump identification information items provided to a plurality of medical pumps arranged bedside in a predetermined order; a data creating step in which image data is created, the image data being used to display pump information items associated with the pump identification information items as a layout that maintains reading order in which the pump identification information items are read; and a displaying step of displaying the image data created in the data creating step.

Using the medical pump monitoring device and the medical pump monitoring method can display the pump information items of a plurality of pumps as the layout reflecting the actual arrangement of the pumps by reading the pump identification information items with the reading terminal in reading order in accordance with the arrangement of the pumps.

### Advantageous Effects of Invention

As described above, according to the present invention, the pump information items of a plurality of pumps can be displayed as the layout reflecting the actual arrangement of the pumps Pn. This can make it easy to link the pump information items on the display image to the pumps actually arranged bedside. As a result, the desired pump is promptly detected from the pumps and an accurate emergency response can be provided.

### Brief Description of Drawings

Fig. 1 is an explanatory diagram of the whole configuration of a medical pump monitoring device of an embodiment and the application environment to which the medical pump monitoring device is applied.
Fig. 2 is a diagram of an exemplary data registration screen used for image data creation in an arithmetic processing unit.
Fig. 3 is an explanatory flowchart (1) of the image data creation in the arithmetic processing unit.
Fig. 4 is an explanatory flowchart (2) of the image data creation in the arithmetic processing unit.
Fig. 5 is a schematic explanatory diagram (1) of a medical pump monitoring method of an embodiment.
Fig. 6 is a schematic explanatory diagram (2) of the medical pump monitoring method of the embodiment.
Fig. 7 is a schematic explanatory diagram (3) of the medical pump monitoring method of the embodiment.
Fig. 8 is a schematic explanatory diagram (4) of the medical pump monitoring method of the embodiment.
Fig. 9 is a schematic explanatory diagram (5) of the medical pump monitoring method of the embodiment.
Fig. 10 is a diagram of a display image (1) on a central monitor of a medical pump monitoring device of an embodiment.
Fig. 11 is a diagram of a display image (2) on the central monitor of the medical pump monitoring device of the embodiment.
Fig. 12 is a diagram of a display image displayed on the bedside monitor of a medical pump monitoring device of the embodiment.

### Description of Embodiments

An embodiment of the medical pump monitoring device and medical pump monitoring method of the present invention will be described in detail hereinafter with reference to the appended drawings. The description of the medical pump monitoring device of the embodiment will be followed by the description of the medical pump monitoring method using the medical pump monitoring device.

### «Medical Pump Monitoring Device»

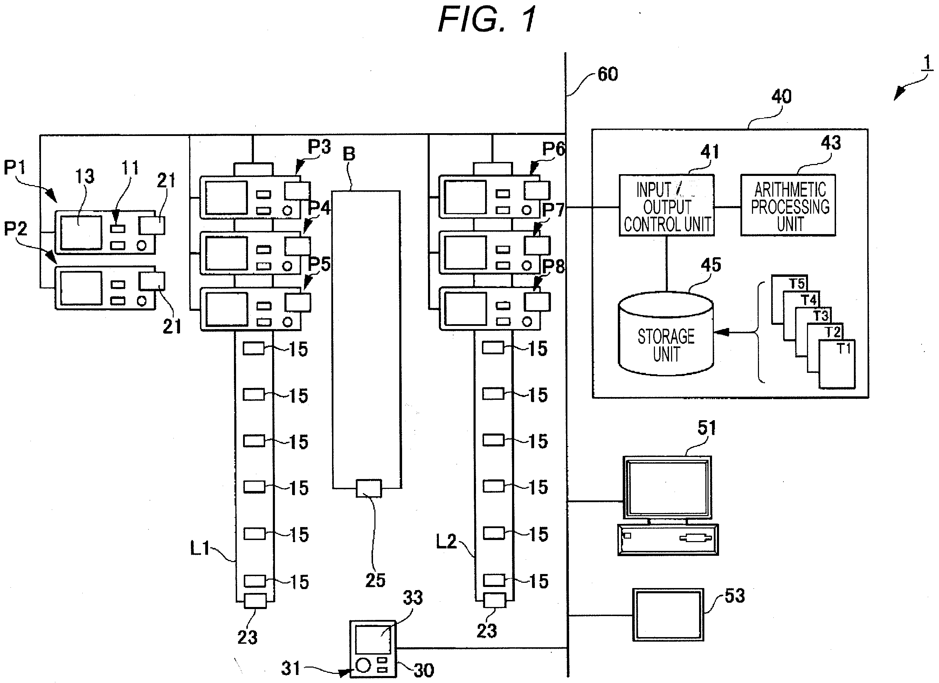

Fig. 1 is a diagram of the whole configuration of an environment in which the medical pump monitoring device of the present invention is used to manage pumps. As illustrated in Fig. 1, a medical pump monitoring device 1 (hereinafter, referred to merely as a "monitoring device 1") is used to manage the drive states of a plurality of medical pumps P1, P2, ... (hereinafter, representatively referred to as "pumps Pn") in an environment in which the pumps Pn are arranged on the side of a bed B. The medical pumps Pn can be placed on pump racks L1, L2, ... (hereinafter, representatively referred to as "racks Lm"), respectively.

In the environment to which the monitoring device 1 is applied, each of the pumps Pn is provided with a pump information storage unit 21. Each of the racks Lm is provided with a rack information storage unit 23. Furthermore, the bed B is provided with a bed information storage unit 25.

The monitoring device 1 includes: a reading terminal 30 for reading information; a server 40 including an input/output control unit 41, an arithmetic processing unit 43, and a storage unit 45; a central monitor 51, and a bedside monitor 53. The configured components can perform network communications, for example, via the communication network provided in the environment. The communication network is, for example, an in-hospital LAN 60. The in-hospital LAN 60 can be either a wireless LAN or a wired LAN.

### <Pumps Pn>

The pumps Pn are all kinds of medical pumps such as a syringe pump and an infusion pump. Each of the pumps Pn includes an operation unit 11 and a display unit 13. The operation unit 11 includes, for example, a power source operation unit, an operation unit configured to start pump operation, a setting operation unit configured to set the flow amount/dosage amount, and a display switching operation unit. The operation unit 11 controls the drive of the pump with a pump drive unit. The display unit 13 is, for example, a liquid crystal panel.

Each of the pumps Pn includes an input/output control unit and a communication unit to the outside (not illustrated) and is connected to the in-hospital LAN 60 so that each of the pumps Pn transmits the pump information including the pump identification information and the drive state of the pump transmitted via the in-hospital LAN 60 to the server 40 using the connection.

Among the pumps Pn, especially, the pump placed on the rack Lm and used includes a short-range communication unit capable of information-communication with the rack Lm. The short-range communication unit performs, for example, infrared communication. The short-range communication unit can communicate with the short-range communication unit provided to the rack Lm, and is configured to transmit the pump identification information that the pump Pn holds to the rack Lm.

### <Pump Information Storage Unit 21>

The pump information storage unit 21 is provided to each of the pumps Pn and holds a pump identification information item associated with each of the pumps Pn on a one-to-one basis. The pump identification information item can be code information such as a bar code. The pump information storage unit 21 holding such code information is, for example, a seal or a plate with a bar code. The pump identification information item held in the pump information storage unit 21 is associated with the pump identification information item that the pump Pn has, and previously stored in a pump identification information table T1 in the storage unit 45 of the server 40.

### <Rack Lm>

Each of the racks Lm is used to place the pumps Pn and has a structure in which the pumps Pn are vertically arranged at predetermiend positions. In the illustrated example, each of the racks L1 and L2 has a structure in which nine pumps Pn can be placed. Three pumps P3 to P5 and three pumps P6 to P8 are placed on the racks L1 and L2, respectively. The three pumps P3 to P5 and the three pumps P6 to P8 are placed on the first, second, and third shelves from the tops of the racks L1 and L2, respectively.

Each of the racks Lm includes an input/output control unit and a communication unit to the outside (not illustrated), and is connected to the in-hospital LAN 60 so that placement position information items of the pumps Pn are transmitted via the in-hospital LAN 60 to the server 40. The rack identification information item together with the pump placement position information items and the pump identification information items of the placed pump are transmitted from the communication unit via the in-hospital LAN 60 to the server 40.

### <Rack Information Storage Unit 23>

The rack information storage unit 23 is provided to each of the racks Lm, and holds a rack identification information item associated with each of the racks Lm on a one-to-one basis. The rack information storage unit 23 is similar to the pump information storage unit 21 and is, for example, a seal or a plate with a bar code. The rack identification information item that the rack information storage unit 23 holds is associated with the rack identification information item that each of the racks Lm has, and is previously stored in a rack identification information table T2 in the storage unit 45 of the server 40.

### <Bed Information Storage Unit 25>

The bed information storage unit 25 is provided to each bed B and holds a bed identification information item associated to each bed B on a one-to-one basis. The bed information storage unit 25 is similar to the pump information storage unit 21 and the rack information storage unit 23, and is, for example, a seal or a plate with a bar code. Note that the bed identification information item can be a patient identification information item given to each patient. In this example, the bed information storage unit 25 is, for example, the patient information storage unit that each patient has. The bed identification information item held in the bed information storage unit 25 is associated with a bed information item used to identify the position at which the bed is located, and is previously stored in a bed identification information table T3 in the storage unit 45 of the server 40.

### <Reading terminal 30>

The reading terminal 30 is configured in the monitoring device 1. The reading terminal 30 is configured to read the information from the pump information storage unit 21, the rack information storage unit 23, and the bed information storage unit 25. Thus, when the pump information storage unit 21, the rack information storage unit 23, and the bed information storage unit 25 have bar codes as the identification information items, the reading terminal 30 is a bar code reader. The reading terminal 30 includes the operation unit 31 and the display unit 33. The operation unit 31 is a unit that inputs, for example, the start information and the completion information to start or complete the reading operation. The display unit 33 is, for example, a liquid crystal display panel.

### <Input/output Control Unit 41 (Server 40)>

The input/output control unit 41 is configured in the monitoring device 1. The input/output control unit 41 collects each identification information item read by the reading terminal 30, the pump information item of each pump Pn transmitted from each of the pumps Pn, and the pump placement position information items and pump identification information items transmitted from the communication unit of the racks Lm, and stores the information items in the storage unit 45.

The input/output control unit 41 creates a new data table in accordance with the information items collected by the input/output control unit 41 and the information items stored in the storage unit 45, and stores the new data table in the storage unit 45.

When collecting a pump information item from a pump Pn, the input/output control unit 41 checks the pump identification information item collected together with the pump information item against the information items stored in the pump identification information table T1 of the storage unit 45. Then, the input/output control unit 41 associates the collected pump information item with the pump identification information item held in the pump information storage unit 21, and stores the information item in a pump information table T4 in the storage unit 45.

When collecting a pump placement position information item and a pump identification information item from a rack Lm, the input/output control unit 41 checks the collected information items against the pump identification information table T1 and the rack identification information table T2 in the storage unit 45. The input/output control unit 41 associates the rack identification information item held in the rack information storage unit 23 with the pump placement position and the pump identification information item, and stores the information item in a pump placement position information table T5 in the storage unit 45. The pump placement position information table T5 indicates which rack Lm and which placement position each of the pumps Pn is placed.

The input/output control unit 41 displays the image data created with the process by the arithmetic processing unit 43 on the central monitor 51 and the bedside monitor 53.

### <Arithmetic Processing Unit 43 (Server 40)>

The arithmetic processing unit 43 is configured in the monitoring device 1. The arithmetic processing unit 43 creates the image data in accordance with the information items collected by the input/output control unit 41 and the information items stored in the storage unit 45 in order to display the pump information items on the central monitor 51 and the bedside monitor 53. The image data creation performed by the arithmetic processing unit 43 will be described in detail below.

### <Storage Unit 45 (Server 40)>

The storage unit 45 is configured in the monitoring device 1. The storage unit 45 stores the information items of the association created by the input/output control unit 41 and another information item. The main information items stored in the storage unit 45 will be described below.

### [Pump Identification Information Table T1]

The pump identification information table T1 is an association table in which the pump identification information item held in a pump information storage unit 21 is associated with the pump identification information item transmitted from a pump Pn.

### [Rack Identification Information Table T2]

The rack identification information table T2 is an association table in which the rack identification information held in a rack information storage unit 23 is associated with the rack identification information transmitted from a rack Lm.

### [Bed Identification Information Table T3]

The bed identification information table T3 is an association table in which the bed identification information item held in a bed information storage unit 25 is associated with a bed information item used to identify, for example, the position where the bed B is located.

### [Pump Information Table T4]

The pump information table T4 is an association table in which the pump identification information item held in a pump information storage unit 21 is associated with the pump information item transmitted from a pump Pn.

### [Pump Placement Position Information Table T5]

The pump placement position information table T5 is an association table in which the rack identification information held in a rack information storage unit 23 is associated with the pump placement position and pump identification information item transmitted from the rack Lm. The pump placement position information table T5 indicates which rack Lm and which placement position each of the pumps Pn is placed.

### [Data Registration Screen Table T6]

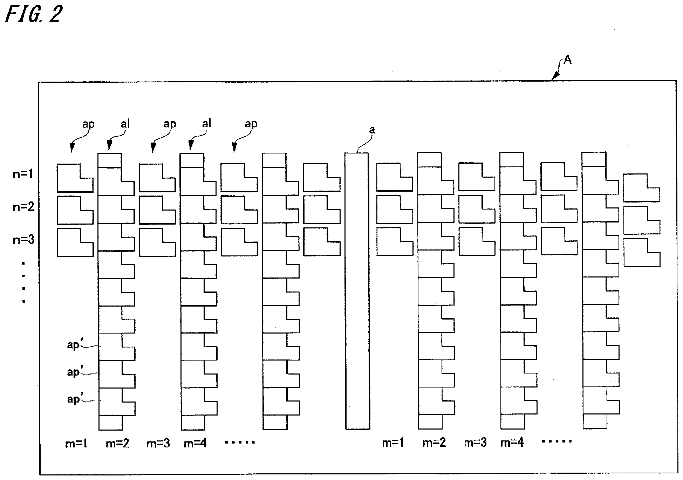

The data registration screen table T6 is a table corresponding to a data registration screen A used to create the image data as illustrated in Fig. 2. The data registration screen A includes pump display units ap, rack information display units al, and a bed display unit a. For example, in the data registration screen A, the bed display unit a is centered, and the pump display units ap displaying the individual pumps and the rack information display units al displaying the racks are alternately arranged on the right and left sides of the bed display unit. In the illustrated example, the pump display units ap in four columns and the rack information display units al in three columns are alternately arranged both in a display area on the left side and in a display area on the right side of the bed display unit a.

For details, the individual pump display units ap are arranged in a column m = 1 from the left end and in rows n = 1 to n = 3. The rack information display unit al is placed in a column m = 2. The individual pump display units ap are arranged in a column m = 3 and in the rows n = 1 to n = 3. Each of the rack information display units al includes a plurality of pump display units ap'. The pump display units ap' are arranged in the direction in which the rows n are arranged.

### <Central Monitor 51 >

The central monitor 51 is configured in the monitoring device 1. The central monitor 51 is configured to manage the pumps Pn arranged for a plurality of beds B, and is located, for example, at a nurses' station. The central monitor 51 is, for example, a display unit of a personal computer. In response to the instructions from the server 40, the bed information item of each of the beds B and the pump information items of the pumps Pn placed on the side of the bed B are displayed based on the image data created by the arithmetic processing unit 43.

### <Bedside Monitor 53>

The bedside monitor 53 is configured in the monitoring device 1. The bedside monitor 53 is placed on the side of the bed B, and can be a monitoring device that only performs display. In response to the instructions from the server 40, the pump information items of the pumps Pn arranged on the side of a bed B are displayed on the bedside monitor 53 based on the image data created by the arithmetic processing unit 43.

### <Operation of Monitoring Device 1>

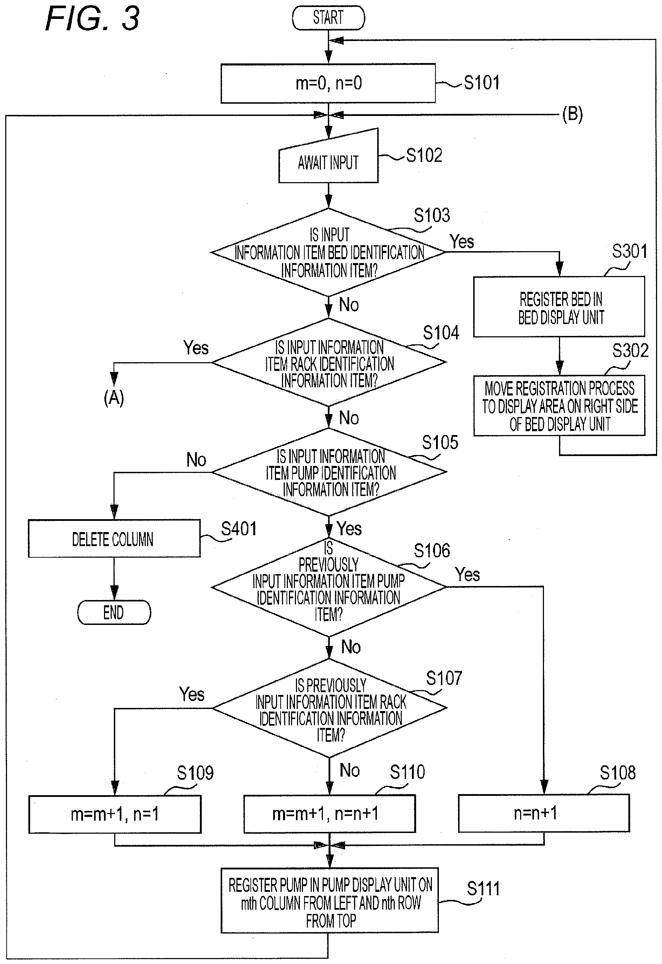

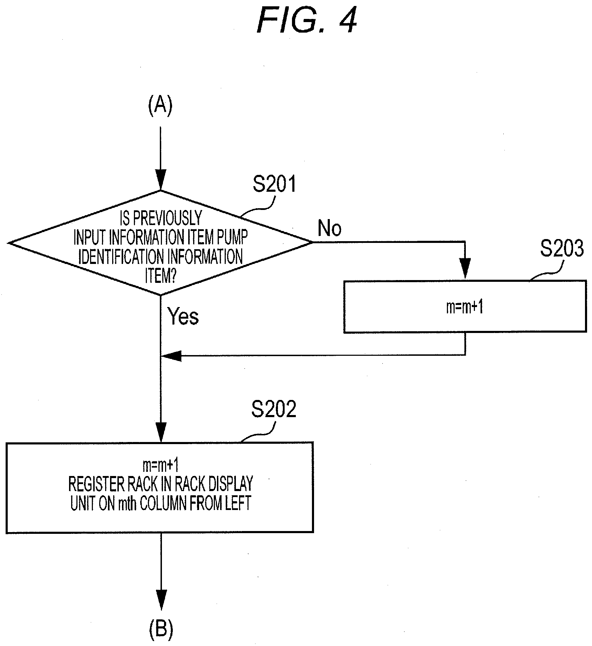

Next, an image data creating process mainly performed by the arithmetic processing unit 43 will be described as the operation of the monitoring device 1 having the configuration described above with reference to Figs. 1 and 2 and along the flowcharts of Figs. 3 and 4.

First, the process starts with the start information input from the reading terminal 30. Subsequently, the arithmetic processing unit 43 initializes the column information m and the row information n so as to set m = 0 and n = 0 (step S101). Next, the arithmetic processing unit 43 awaits the input of an identification information item from the reading terminal 30 and another information item (step S102).

After that, the arithmetic processing unit 43 determines which identification information item is the information item input from the reading terminal 30 (steps S103 to S105). When the arithmetic processing unit 43 determines in step S103 that the input information item is a bed identification information item (Yes), the process goes to step S301. When the arithmetic processing unit 43 determines in step S104 that the input information item is a rack identification information item (Yes), the process goes to step S201 illustrated in Fig. 4. When the arithmetic processing unit 43 determines in step S105 that the input information item is a pump identification information item (Yes), the process goes to step S106. When the arithmetic processing unit 43 determines that the input information is not any one of the information items, the arithmetic processing unit 43 determines that the input information item is the completion information. Then, the process goes from step S105 to step S401.

When it is determined that the input information item is a pump identification information item and the process goes to step S106, the arithmetic processing unit 43 determined whether the previous input is a pump identification information item. When the arithmetic processing unit 43 determines that the previous input is a pump identification information item (Yes), the process goes to step S108 and the arithmetic processing unit 43 rewrites the row information n as n = n + 1. On the other hand, when the arithmetic processing unit 43 determines in step S106 that the previous input is not a pump identification information item (No), the process goes to step S107. The arithmetic processing unit 43 determines in step S107 whether the previous input is a rack identification information item. When the arithmetic processing unit 43 determines that the previous input information item is a rack identification information item (Yes), the process goes to step S109 and the arithmetic processing unit 43 rewrites the column information m as m = m + 1 and the row information as n = 1. When the arithmetic processing unit 43 determines that the previous input information item is not a rack identification information item (No), the process goes to step S110 and the arithmetic processing unit 43 rewrites the column information m as m = m + 1 and the row information as n = n + 1.

After that, in accordance with the column information m and row information n rewritten in steps S108 to S110, the arithmetic processing unit 43 associates the input pump identification information item with the display unit in the mth column from the left and the nth row from the top in the display area on the left side of the bed display unit a in the table for creating the data registration screen A described above with reference to Fig. 2, and registers the associated information item in the display unit (step S111).

On the other hand, when the arithmetic processing unit 43 determines in step S104 that a rack identification information item is input and the process goes to step S201 illustrated in Fig. 4, the arithmetic processing unit 43 determines whether the previous input is a pump identification information item. When the arithmetic processing unit 43 determines that the previous input information item is a pump identification information item (Yes), the process goes to step S202. On the other hand, when the arithmetic processing unit 43 determines that the input information item is not a pump identification information item (No), the process goes to step S203 and the arithmetic processing unit 43 rewrites the column information m as m = m + 1. Then, the process goes to step S202.

Next, the arithmetic processing unit 43 rewrites the column information m as m = m + 1 in step S202, and registers a rack in the rack information display unit al in the column of the rewritten column information in the display area on the left side of the bed display unit a in the table for creating the data registration screen A described above with reference to Fig. 2. In this example, the pump identification information item of each pump is registered in each row corresponding to the pump placement position associated with the rack identification information item. After that, the process returns to step S102 illustrated in Fig. 3 and the arithmetic processing unit 43 awaits input.

When the arithmetic processing unit 43 determines in step S103 that a bed identification information item is input and the process goes to step S301, the arithmetic processing unit 43 registers the bed identification information item in the bed display unit a in the table for creating the data registration screen A described above with reference to Fig. 2.

After that, the arithmetic processing unit 43 performs a process for moving the registration area to a display area of the right side of the bed display unit a in the data registration screen A for registration in the subsequent registration processes (step S302). Next, the process returns to step 101 and the arithmetic processing unit 43 initializes the column information m and the row information n so as to set m = 0 and n = 0 (step S101).

Subsequently, the process for registering a pump identification information item is performed in the display area on the right side of the bed display unit a in the data registration screen A.

When the arithmetic processing unit 43 determines in step S105 that the input information item is not any one of the identification information items (and is the completion information) and the process goes to step S401, the arithmetic processing unit 43 deletes the display units in the column in which an information item is not registered, and terminates the process.

As described above, the arithmetic processing unit 43 is configured to rewrite the column information m and the row information n depending on the type of the previously input information item when the arithmetic processing unit 43 registers the pump information item associated with the input identification information item in the data registration screen A set as illustrated in Fig. 2. By this configuration, a process for registering the next pump information item in the position next to the position of the previously input pump information item in a direction in which the column information m or the row information n increases is repeated. As a result of the repetition, the pump information items are registered in the data registration screen A as the layout reflecting the reading order in which the identification information items are read. Thus, the reading order in which the reading terminal 30 reads the identification information items is arranged in the order in which the pumps Pn, the racks Lm, and the bed B are arranged. This enables the display of the pump information items of the pumps Pn as the layout reflecting the actual arrangement of the pumps Pn.

### «Medical Pump Monitoring Method»

Next, a medical pump monitoring method using the medical pump monitoring device and performed in the application environment described above will be described in detail. Herein, the application environment illustrated in Fig. 1 is cited as an example. A medical pump monitoring method in the application environment will be described.

### <Reading Step>

In a reading step, the reading terminal 30 reads the pump identification information item in the pump information storage unit 21 provided to a pump Pn, the rack identification information item in the rack information storage unit 23 provided to a rack Lm, and the bed identification information item in the bed information storage unit 25 installed on a bed B in a predetermined order.

For the reading of the identification information items by the reading terminal 30, it is important that the identification information items are read sequentially from a first end to a second end in accordance with the arrangement of the pumps Pn, the racks Lm, and the bed B. As an example, the reading terminal 30 reads the identification information items sequentially from the left side to right side of the bed B and from the top to the bottom.

Specifically, first, the pump identification information items of the pumps P1 and P2 longitudinally arranged on the left end in the environment in which the pumps are managed as illustrated in Fig. 1 are read sequentially from the top to the bottom in the order from the pump P1 to the pump P2. Next, the rack identification information item of a Rack L1 placed on the right side of the pumps P1 and P2 is read. Next, the bed identification information item of the bed B placed on the right side of the rack L1 is read. Next, the rack identification information item of a rack L2 placed on the right side of the bed B.

### <Data Creating Step>

In a data creating step, image data used to display drive information items is created as the layout that maintains the reading order in which the identification information items are read in the reading step. In the data creating step, the arithmetic processing unit 43 of the monitoring device 1 creates the image data used to display the drive information items of the pumps Pn, the bed position information item, and the rack position information items as the layout that maintains the reading order in which the pump identification information items, the rack identification information items, and the bed identification information item are read by creating the image data in the process described above with reference to the flowcharts of Figs. 3 and 4. The arithmetic processing unit 43 creates the image data used to display the drive information item of each of the medical pumps Pn placed on a rack Lm as the layout that maintains the placement positions at which the pumps are placed on the racks Lm. Meanwhile, the arithmetic processing unit 43 creates the image data in accordance with the placement position information items of the medical pumps Pn on the rack Lm associated with the rack identification information item.

The image data is created in the data creating step as described above is as follows.

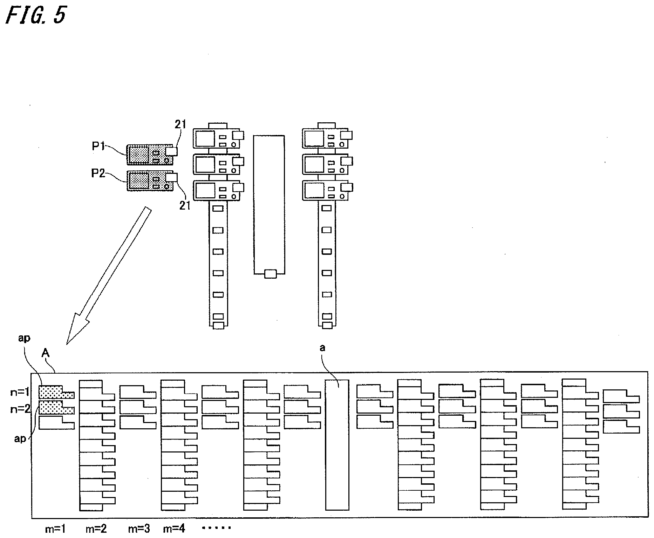

First, the reading terminal 30 reads the pump identification information item from the pump information storage unit 21 of the pump P1 of the pumps P1 and P2 longitudinally arranged on the leftmost end of the area of the left side of the bed B as illustrated in Fig. 5 in the previous reading step. Thus, the arithmetic processing unit 43 registers the pump in the pump display unit ap in the column m = 1 and the row n = 1 in the display area on the left side of the bed display unit a in the data registration screen A in accordance with the flowchart.

Then, the reading terminal 30 reads the pump identification information item from the pump information storage unit 21 of the pump P2 after the pump P1. This causes the arithmetic processing unit 43 to register the pump identification information item of the pump P2 in accordance with the flowchart. In other words, the arithmetic processing unit 43 associates the input pump identification information item of the pump P2 with the pump display unit ap in the column m = 1 and the row n =2 next to the position of the pump display unit ap in the column m = 1 and the row n =1 in which the pump information item of the pump P1 is previously registered in the display area on the left side of the bed display unit a in the data registration screen A, and register the information item.

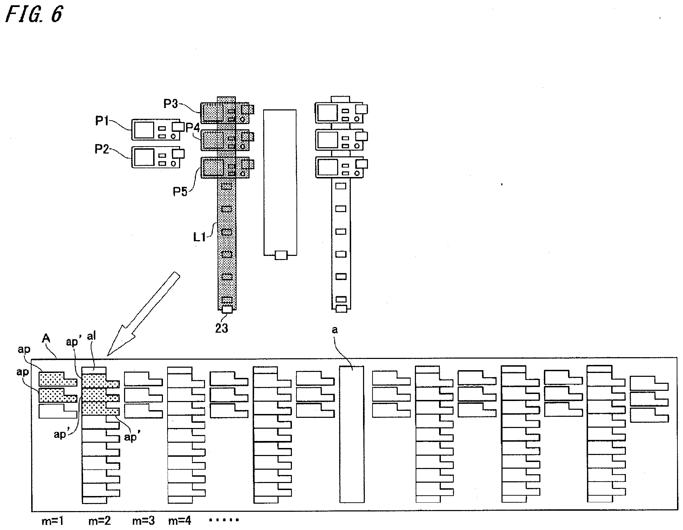

Next, the reading terminal 30 reads the rack identification information item from the rack information storage unit 23 of the rack L1 on the right side of the pumps P1 and P2 as illustrated in Fig. 6 in the previous reading step. This causes the arithmetic processing unit 43 to register the pump identification information items in each row corresponding to the pump placement positions associated with the input rack identification information item in the rack information display unit al in the column m = 2 in the display area on the left side of the bed display unit a in the data registration screen A in accordance with the flowchart.

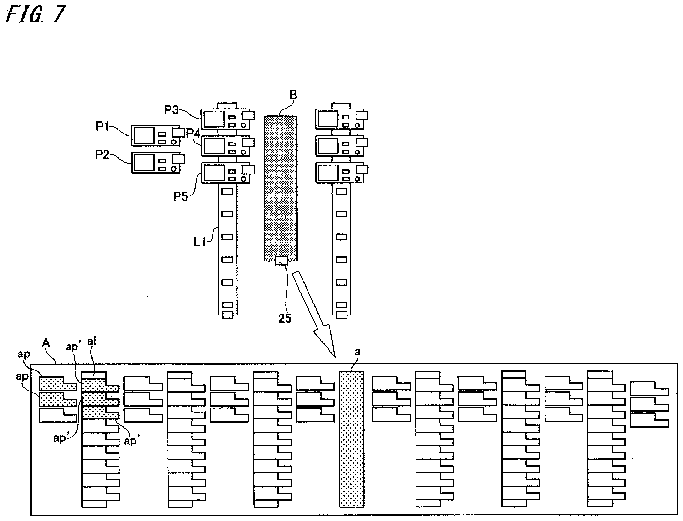

Next, the reading terminal 30 reads the bed identification information item from the bed information storage unit 25 of the bed B as illustrated in Fig. 7 in the previous reading step. This causes the arithmetic processing unit 43 to register the bed identification information item in the bed display unit a in the table for creating the data registration screen A in accordance with the flowchart.

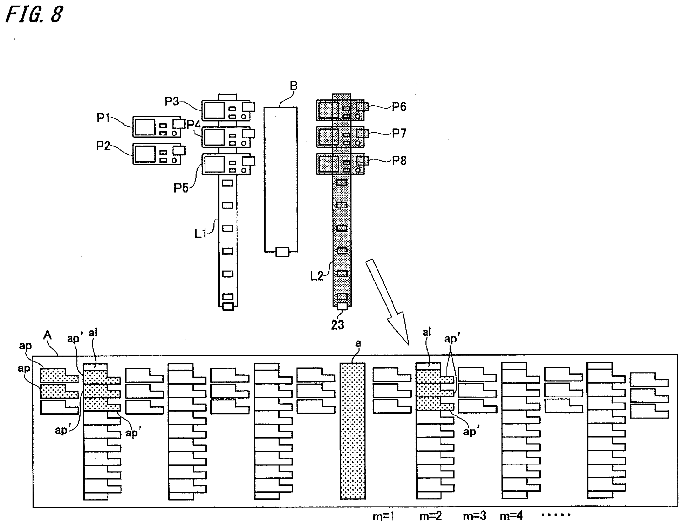

Next, the reading terminal 30 reads the rack identification information item in the rack L2 on the right side of the bed B as illustrated in Fig. 8 in the previous reading step. This causes the arithmetic processing unit 43 to move the registration area to the display area on the right side of the bed display unit a in the data registration screen A in order to perform registration in accordance with the flowchart. The arithmetic processing unit 43 registers each pump identification information item in each placement position of the pumps associated with the input rack identification information item in the rack information display unit al in the column m = 2 in the display area on the right side of the bed display unit a.

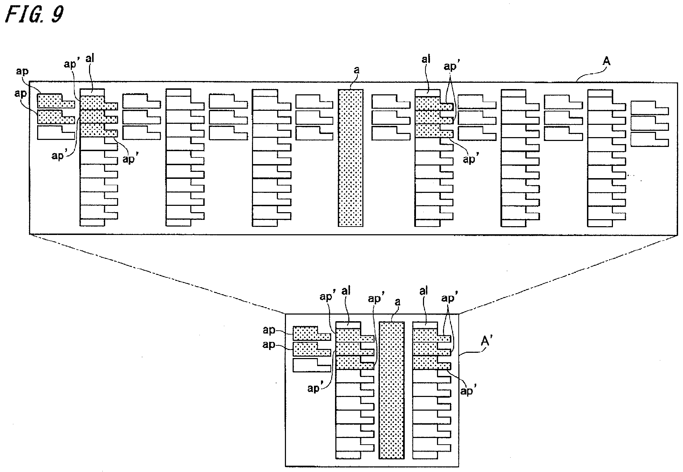

When the reading is completed in the reading step and the reading terminal 30 inputs the completion information, the arithmetic processing unit 43 deletes the display of the column in which an information item is not registered from the data registration screen A. With this deletion, the arithmetic processing unit 43 creates the image data of a registration screen A' only including the information items registered in the left columns as illustrated in Fig. 9.

### <Displaying Step>

In a displaying step, the image data created described above in the data creating step is displayed on the central monitor 51 and the bedside monitor 53.

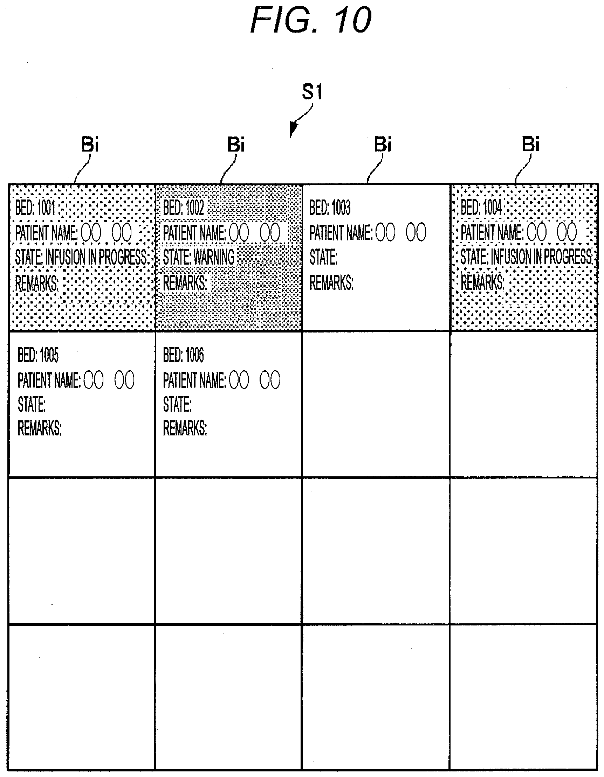

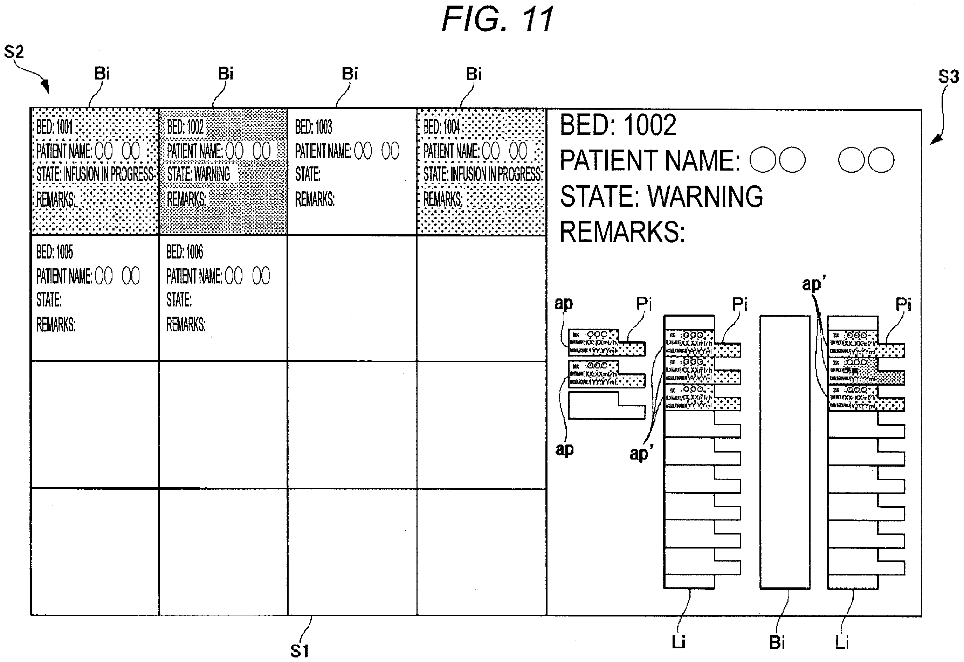

Fig. 10 illustrates a display image S1 on the central monitor 51 of the monitoring device 1. As illustrated in the drawing, the display image S1 on the central monitor 51 displays the bed information items of the beds B managed on the central monitor 51. Each bed display Bi displays, for example, the patient information. When a bed display Bi (a bed: 1002 in this example) is designated, the central monitor 51 displays, as the display image S2 illustrated in Fig. 11, a detailed information image S3 associated with the designated bed display Bi together with the reduced display image S1.

The detailed information image S3 displays the image corresponding to the created registration screen A', and displays the pump information items of the pumps Pn in the pump display units ap and ap' in which the pumps Pn are registered. Each pump information item Pi is, for example, the name of the drug, the flow amount, and the accumulation amount. When the arithmetic processing unit 43 determines in accordance with the pump information item transmitted from each of the pumps Pn that a pump Pn is not in a normal drive state, the pump information item Pi and bed display Bi of the pumps Pn can be displayed in reversed colors.

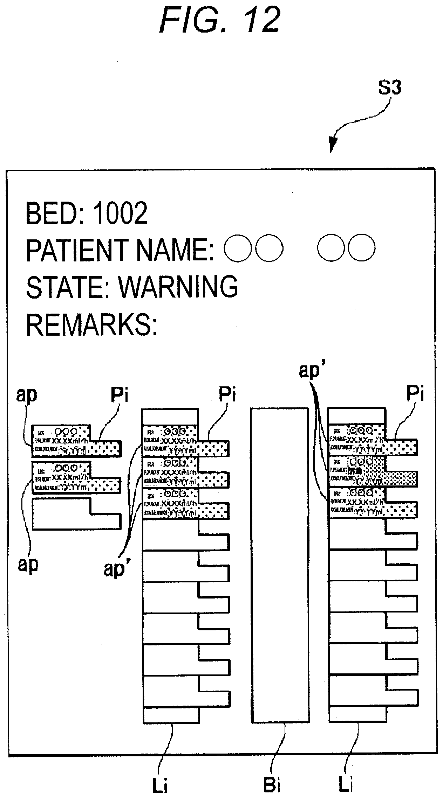

Note that the bedside monitor 53 displays the detailed information image S3 as illustrated in Fig. 12. The detailed information image S3 is the same as the detailed information image S3 displayed on the central monitor 51.

Especially, the detailed information image S3 in Figs. 11 and 12 displays the pump information items Pi of the pumps Pn in an arrangement reflecting the actual arrangement of the pumps Pn. In other words, the detailed information image S3 is the display image based on the image data created in the previous data creating step.

The arrangement of the pump information items Pi, the rack information displays Li, and the bed display Bi in the detailed information image S3 is the arrangement of the pumps Pn, the racks Lm, and the bed B in Fig. 1. This is because the reading terminal 30 reads the identification information items in the predetermined order described above and the arithmetic processing unit 43 creates the image data so as to display the data related to the pumps Pn, the racks Lm, and the bed B as the layout that maintains the reading order.

The rack information display Li also includes the pump information items Pi. The arrangement of the pump information items Pi configured in the rack information display Li corresponds to the arrangement of the pumps Pn placed on the racks Lm in Fig. 1. This is because the arithmetic processing unit 43 creates the image data so as to display the data associated with each of the pumps Pn as the layout that maintains the placement order in accordance with the rack identification information item read in the reading step.

As described above, the monitoring device 1 and monitoring method of the present embodiment displays the pump information items Pi associated with the pump identification information items as the layout that maintains the reading order in which the reading terminal 30 reads the pump identification information items. Thus, arranging the reading order in which the reading terminal 30 reads the pump identification information items as the order in which the pumps Pn are arranged allows for the display of the pump information items Pi of the pumps Pn as the layout reflecting the actual arrangement of the pumps Pn.

This enables easy link of the pump information items Pi in the display image on the central monitor 51 or the bedside monitor 53 to the pumps Pn actually arranged bedside. As a result, the desired pump Pn of which pump information item Pi is confirmed on the display image can promptly be detected among the pumps Pn. Accurate medical treatment can be given even in an emergency response.

Note that the present invention is not limited to the embodiments and exemplary variations described above with reference to the drawings. The present invention can include various variations without departing from the gist of the present invention described in claims.

For example, the arithmetic processing unit 43 of the medical pump monitoring device can create the image data while giving addresses to the sequentially read identification information items. In this example, an address is given also to the bed identification information item.

According to the medical pump monitoring method, the reading order in which the reading terminal 30 reads the identification information items is not limited to the order described above, and can be any order in which the identification information items are read sequentially from an end to the other end. Thus, the identification information items can be read from the right end to the left end. The arithmetic processing unit 43 performs a left-right reversal of the process described with reference to the flowcharts of Figs. 3 and 4.

### Reference Signs List

- 1: Medical pump monitoring device
- 30: Reading terminal
- 43: Arithmetic processing unit
- B: Bed
- Lm: Pump rack
- Pn, P1, P2: Medical pump

## Claims

1. A medical pump monitoring device configured to manage a plurality of medical pumps arranged bedside, the medical pump monitoring device comprising:
a reading terminal configured to sequentially read pump identification information items provided to the medical pumps; and
an arithmetic processing unit configured to create image data used to display pump information items associated with the pump identification information items as a layout that maintains reading order in which the reading terminal reads the pump identification information items.

2. The medical pump monitoring device according to claim 1, wherein
the reading terminal reads a bed identification information item corresponding to each bed, and
the arithmetic processing unit creates image data used to display the bed position information item together with the pump information items as a layout that maintains reading order in which the pump identification information items and the bed identification information item are read.

3. The medical pump monitoring device according to claim 1 or 2, wherein
the reading terminal reads a rack identification information item provided to a pump rack on which a plurality of medical pumps is placed, and
in accordance with pump placement position information items and pump information items associated with the rack identification information item, the arithmetic processing unit creates image data used to display the pump information items as a layout that maintains reading order in which the reading terminal reads the pump identification information items and the rack identification information item, and also maintains placement order in accordance with the pump placement position information items.

4. A medical pump monitoring method comprising:
a reading step of reading pump identification information items provided to a plurality of medical pumps arranged bedside in a predetermined order;
a data creating step in which image data is created, the image data being used to display pump information items associated with the pump identification information items as a layout that maintains reading order in which the pump identification information items are read; and
a displaying step of displaying the image data created in the data creating step.

5. The medical pump monitoring method according to claim 4, wherein
in the reading step, a bed identification information item corresponding to a bed placed together with a plurality of medical pumps is read in the predetermined order, similarly to the pump identification information items, and
in the data creating step, image data is created, the image data being used to display a bed position information item together with the pump information items as a layout that maintains reading order in which the pump identification information items and the bed identification information item are read.

6. The medical pump monitoring method according to claim 4 or 5, wherein
in the reading step, a rack identification information item provided to a pump rack on which a plurality of medical pumps is placed is read in the predetermined order, similarly to the pump identification information items, and
in the data creating step, image data is created in accordance with pump placement position information items and pump information items associated with the rack identification information item, the image data being used to display the pump information items as a layout that maintains reading order in which the pump identification information items and the rack identification information item are read and also maintains placement order in accordance with the pump placement position information items.

7. The medical pump monitoring method according to any one of claims 4 to 6, wherein
in the reading step, the pump identification information items are read sequentially from a first end to a second end in accordance with arrangement of the medical pumps.

8. The medical pump monitoring method according to any one of claims 4 to 7, wherein
in the data creating step, pump information items associated with subsequently read pump identification information items, respectively, are registered from a position next to a position in which a pump information item associated with a pump identification information item read immediately before the subsequently read pump identification information item in the reading step is registered in a predetermined direction.
